# EUROPÄISCHE PATENTANMELDUNG

(11) **EP 3 081 246 A1**
(43) Veröffentlichungstag der Anmeldung: **19.10.2016**
(21) Anmeldenummer: 15163311.2
(22) Anmeldetag: 13.04.2015
(51) Int. Cl.: A61M 1/10

(54) **PUMPE SOWIE VERFAHREN ZUM BETRIEB EINER PUMPE FÜR FLÜSSIGKEITEN**

(71) Anmelder: Berlin Heart GmbH, 12247 Berlin (DE)
(72) Erfinder: Kallenbach, Sebastian, 13353 Berlin (DE); Rodemerk, Aaron, 14482 Potsdam (DE); Strommenger, Daniel, 12161 Berlin (DE); Wisniewski, Adrian, 10963 Berlin (DE)
(74) Vertreter: Pfenning, Meinig & Partner mbB

(57) **Zusammenfassung**

Die Erfindung bezieht sich auf ein Verfahren zum Betrieb einer Pumpe (6) mit einem Rotor (6b, 6c), insbesondere einer Blutpumpe, zur Förderung von Flüssigkeiten, bei dem der Volumenstrom durch die Pumpe und die Druckdifferenz über der Pumpe ermittelt wird, wobei wenigstens ein erster und insbesondere ein zweiter Betriebsparameter einer ersten Gruppe von Betriebsparametern erfasst und in Abhängigkeit von dem Wert des ersten und insbesondere auch dem Wert eines zweiten Betriebsparameters der ersten Gruppe der Volumenstrom durch die Pumpe und die Druckdifferenz über die Pumpe aus erfassten Werten der Betriebsparameter entweder aus der ersten Gruppe von Betriebsparametern ermittelt wird oder zur Ermittlung des Volumenstroms und der Druckdifferenz über die Pumpe Werte eines anderen Satzes von Betriebsparametern, insbesondere wenigstens eines zusätzlichen Betriebsparameters, berücksichtigt werden. Durch Verwendung verschiedener Ermittlungsverfahren für den Volumenstrom durch die Pumpe lässt sich diese Größe ebenso wie auch die Druckdifferenz über der Pumpe über das gesamte Kennlinienfeld mit der erforderlichen Genauigkeit bestimmen, wobei in verschiedenen Kennlinienfeldbereichen zwischen den verschiedenen Verfahren gewechselt wird.

## Beschreibung

Die Erfindung liegt auf dem Gebiet des Maschinenbaus und der Elektrotechnik und ist insbesondere auf dem Gebiet der Medizintechnik mit Vorteil verwendbar. Insbesondere bezieht sich die Erfindung auf eine Pumpe für Flüssigkeiten im medizinischen Bereich, bei der im Betrieb der Volumendurchsatz der Pumpe und/oder eine durch die Pumpe erzeugte Druckdifferenz erfasst werden soll.

Im medizinischen Bereich werden Flüssigkeitspumpen für verschiedene Zwecke eingesetzt, einerseits zur Förderung von Körperflüssigkeiten, insbesondere z. B. Blut, und andererseits zur Förderung von körperfremden Flüssigkeiten, beispielsweise pharmazeutisch wirksamen Flüssigkeiten, die innerhalb des, zum oder vom Körper eines Patienten gefördert werden sollen. Besonders für Blutpumpen, die innerhalb des Patientenkörpers oder auch teilweise außerhalb des Patientenkörpers Blut fördern und die insbesondere als VAD-Pumpen (ventricular assist device) eingesetzt werden, bestehen hohe Anforderungen an die Kontrolle und Steuerung, um mit möglichst großer Genauigkeit und Zuverlässigkeit einen Volumendurchsatz der Pumpe und/oder eine Druckdifferenz messen bzw. einstellen zu können.

Üblicherweise kann für VAD-Blutpumpen, die das Blut beispielsweise vom linken Ventrikel in die Aorta befördern können, oder auch für andere Blutpumpen, beispielsweise RVAD-Pumpen, aus der Drehzahl eines Pumpenrotors und der Druckdifferenz über der Pumpe der Volumendurchsatz aufgrund eines Kennlinienfeldes der Pumpe bestimmt werden. Erfahrungen haben jedoch gezeigt, dass ein Kennlinienfeld, das üblicherweise Werte von zwei oder mehr Betriebsparametern miteinander verknüpft, nicht für den gesamten Einsatzbereich einer solchen Pumpe mit gleicher Genauigkeit und Zuverlässigkeit verwendbar ist.

Dabei sind grundsätzlich verschiedene Methoden zur Ermittlung des Volumendurchsatzes einer Pumpe oder der Druckdifferenz aus verschiedenen Sätzen von Betriebsparametern bekannt.

Der vorliegenden Erfindung liegt vor dem Hintergrund des Standes der Technik die Aufgabe zugrunde, im Betrieb über einen möglichst großen Bereich von möglichen Betriebsparametern eine zuverlässige Ermittlung eines oder mehrerer weiterer Betriebsparameter, insbesondere des Volumenstroms der Pumpe, zu ermöglichen.

Die Aufgabe wird mit den Merkmalen der Erfindung gemäß Patentanspruch 1 durch ein Betriebsverfahren gelöst.

Der Patentanspruch 10 charakterisiert ein erfindungsgemäßes Verfahren zur Ermittlung von Kennlinien der Pumpe, und der unabhängige Patentanspruch 11 bezieht sich auf eine erfindungsgemäße Pumpe mit Einrichtungen zur Erfassung bestimmter Betriebsparameter. Die Patentansprüche 12 bis 15 bezeichnen Ausgestaltungen der Pumpe.

Demgemäß bezieht sich die Erfindung zunächst auf ein Verfahren zum Betrieb einer Pumpe mit einem Rotor zur Förderung von Flüssigkeiten, insbesondere einer Blutpumpe, bei dem der Volumenstrom durch die Pumpe und insbesondere die Druckdifferenz über der Pumpe ermittelt wird, wobei wenigstens ein erster und insbesondere ein zweiter Betriebsparameter einer ersten Gruppe von Betriebsparametern erfasst und in Abhängigkeit von dem Wert des ersten und insbesondere auch dem Wert eines zweiten Betriebsparameters der ersten Gruppe der Volumenstrom durch die Pumpe und insbesondere die Druckdifferenz aus erfassten Werten der Betriebsparameter entweder aus der ersten Gruppe von Betriebsparametern ermittelt wird oder zur Ermittlung des Volumenstroms und insbesondere der Druckdifferenz Werte eines anderen Satzes von Betriebsparametern, insbesondere wenigstens eines zusätzlichen Betriebsparameters, berücksichtigt werden.

Der Erfindung liegt der Gedanke zugrunde, dass bei einer Pumpe der genannten Art durchaus mehrere verschiedene Verfahren zur Ermittlung des Volumenstroms durch die Pumpe und insbesondere der Druckdifferenz über der Pumpe verwendet werden können. Dabei werden die Werte von Betriebsparametern der Pumpe derart aufgeteilt, dass für bestimmte Werte von ausgewählten Betriebsparametern ein erstes Verfahren zur Ermittlung des Volumenstroms und insbesondere der Druckdifferenz verwendet wird und dass für andere Werte von Betriebsparametern ein hiervon verschiedenes Verfahren zur Ermittlung des Volumenstroms und insbesondere der Druckdifferenz unter Verwendung eines zusätzlichen Betriebsparameters verwendet wird. Es findet somit eine Aufteilung eines Kennlinienfeldes der Pumpe nach bestimmten Werten der Betriebsparameter statt, wobei für verschiedene Bereiche des Kennlinienfeldes unterschiedliche Ermittlungsverfahren eingesetzt werden können. Hierdurch kann für jeden Bereich des Kennlinienfeldes unter Berücksichtigung unterschiedlicher Sätze von Betriebsparametern, die sich überschneiden können, beispielsweise das sensitivste Verfahren oder das Verfahren eingesetzt werden, für das die jeweils benötigten Werte der Betriebsparameter am einfachsten zu erfassen sind.

Ein zweites Verfahren zur Ermittlung des Volumenstroms und/oder der Druckdifferenz über der Pumpe kann dazu vorsehen, dass ein zusätzlicher Betriebsparameter berücksichtigt wird, der bei einem ersten Verfahren nicht berücksichtigt wird. Dabei kann vorgesehen sein, dass bei dem zweiten Verfahren sämtliche Betriebsparameter Berücksichtigung finden, die auch bei dem ersten Verfahren berücksichtigt werden, oder dass nur ein Teil oder gar keiner der Betriebsparameter berücksichtigt werden, die bei dem ersten Verfahren berücksichtigt werden. Wichtig ist, dass sich die bei dem ersten und dem zweiten Verfahren zur Ermittlung des Volumenstroms und/oder der Druckdifferenz berücksichtigten Betriebsparameter in wenigstens einem Betriebsparameter unterscheiden.

Es ist auch denkbar, in weiteren Bereichen des Kennlinienfeldes zusätzliche Verfahren einzusetzen, die wiederum andere Betriebsparameter berücksichtigen als das erste und das zweite Verfahren.

Zudem kann auch vorgesehen sein, in bestimmten Bereichen des Kennlinienfeldes mehrere Verfahren gleichzeitig einzusetzen, um die so ermittelten und gegebenenfalls leicht differierenden Werte aneinander anzupassen und so den Fehler der Berechnung zu minimieren.

Eine vorteilhafte Ausgestaltung der Erfindung sieht vor, dass die erste Gruppe von Betriebsparametern die erfasste Drehzahl der Pumpe enthält. Die Drehzahl der Pumpe ist bei einer Rotorpumpe grundsätzlich eine aussagekräftige Größe für die Bestimmung des Volumenstroms.

Vorteilhaft kann zudem vorgesehen sein, dass die erste Gruppe von Betriebsparametern die erfasste Druckdifferenz über den Rotor enthält. Dabei kann insbesondere vorgesehen sein, dass die erste Gruppe von Betriebsparametern die erfasste Kraft auf ein Rotorlager, insbesondere ein Axiallager der Pumpe (Lagerspannung), enthält.

Auch durch die Erfassung der Drehzahl und der Druckdifferenz über der Pumpe, d. h. der Differenz des Drucks vor der Pumpe am Pumpeneinlass und hinter der Pumpe am Pumpenauslass, kann üblicherweise in einem großen Bereich der Betriebszustände der Volumenstrom durch die Pumpe zuverlässig bestimmt werden.

In vielen Fällen wird als Pumpe eine Rotorpumpe eingesetzt, deren Rotor die Flüssigkeit in axialer Richtung fördert. Als Reaktionskraft der geförderten Flüssigkeit entsteht eine auf den Rotor wirkende Axialkraft, die in einem entsprechenden Axiallager aufgefangen wird. Die auf dieses Lager wirkende Axialkraft kann in vielen Fällen erfasst und der Bestimmung der Druckdifferenz über der Pumpe zugrunde gelegt werden.

Insbesondere werden für medizinische Pumpen oft magnetische Axiallager eingesetzt, um einerseits die Reibung gering zu halten, andererseits möglichst wenig Abrieb zu erzeugen und zudem die Moleküle der geförderten organischen Flüssigkeit möglichst wenig durch mechanische Einwirkung zu schädigen. Bei derartigen Magnetlagern ist die wirkende Axialkraft durch Messung der in den Magneten wirkenden Kraft in einfacher Weise ermittelbar. Insbesondere bei geregelten Magnetlagern wird die Position des Rotors üblicherweise erfasst und derart geregelt, dass durch einen Strom eines stationären Elektromagneten die magnetische Axialkraft im Lager als Gegenkraft der axialen Belastung des Rotors erzeugt wird. Durch die Messung der im Wirbelstromsensor induzierten Spannung lässt sich Rotorposition in einfacher Weise bestimmen. Diese Spannung des Wirbelstromsensors ist damit eine die Axialkraft auf den Rotor und damit die Druckdifferenz über den Rotor repräsentierende Größe.

Eine besondere Ausgestaltung der Erfindung kann vorsehen, dass für die Entscheidung, welche Betriebsparameter zur Ermittlung des Volumenstroms durch die Pumpe berücksichtigt werden, ausschließlich die Drehzahl der Pumpe und die Axialkraft auf den Pumpenrotor herangezogen werden.

Zusätzlich kann in einer besonderen Gestaltung auch die Temperatur der geförderten Flüssigkeit mit herangezogen werden.

Es kann auch vorteilhaft vorgesehen sein, dass die erste Gruppe von Betriebsparametern enthält: die Temperatur der Flüssigkeit und/oder den absoluten Druck der Flüssigkeit vor oder hinter der Pumpe und/oder das auf den Rotor der Pumpe wirkende Drehmoment, das insbesondere mittels einer den Speisestrom eines den Rotor antreibenden Elektromotors repräsentierenden Größe bestimmt wird.

Somit können auch beispielsweise die Drehzahl des Rotors, die Axialkraft auf den Rotor und das Drehmoment, das auf den Rotor wirkt, als Betriebsparameter für die Entscheidung herangezogen werden, welches Messverfahren für die Ermittlung des Volumenstromes verwendet wird und welche Betriebsparameter hierfür berücksichtigt werden.

Eine andere Variante kann vorsehen, dass für die Entscheidung, welche Betriebsparameter berücksichtigt werden, die Drehzahl des Rotors, die Axialkraft auf den Rotor und ein absoluter Druckwert der Flüssigkeit vor oder hinter der Pumpe herangezogen werden.

Die genannten beiden Varianten der Erfindung können beispielsweise auch vorsehen, dass jeweils die genannten Betriebsparameter ausschließlich für die Entscheidung herangezogen, welches Messverfahren angewendet wird, und dass die genannten Parameter auch ausschließlich für die Durchführung der Bestimmung des Volumendurchsatzes herangezogen werden.

Eine weitere vorteilhafte Ausgestaltung der Erfindung sieht vor, dass ein zusätzlicher Parameter durch den absoluten Druck der Flüssigkeit vor oder hinter der Pumpe und/oder das auf den Rotor der Pumpe wirkende Drehmoment gebildet ist, das insbesondere mittels einer den Phasenstrom eines den Rotor antreibenden Elektromotors, insbesondere BLDC-Motors (bürstenlosen DC-Motors), repräsentierenden Größe bestimmt wird.

Je nachdem, welche Betriebsparameter bei dem ersten Ermittlungsverfahren berücksichtigt werden, kann für das hiervon verschiedene Ermittlungsverfahren ein weiterer Betriebsparameter berücksichtigt werden, der bei dem ersten Messverfahren nicht berücksichtigt wurde. Damit kann in dem entsprechenden Bereich des Kennlinienfeldes die Schwäche des ersten Ermittlungsverfahrens durch Berücksichtigung wenigstens eines zusätzlichen Betriebsparameters ausgeglichen werden, wobei gleichzeitig auch ein anderer Betriebsparameter bei der Auswertung weggelassen werden kann.

Ein solcher zusätzlicher Betriebsparameter kann beispielsweise auch die Temperatur der zu fördernden Flüssigkeit sein.

In einer konkreten Ausgestaltung der Erfindung kann beispielsweise vorgesehen sein, dass die Druckdifferenz über dem Rotor (insbesondere repräsentiert durch die erfasste Lagerposition) und die Drehzahl der Pumpe erfasst und in Abhängigkeit von wenigstens einem der erfassten Werte der Volumenstrom durch die Pumpe entweder aus den erfassten Werten dieser beiden Betriebsparameter ermittelt wird oder zur Ermittlung des Volumenstroms und insbesondere der Druckdifferenz über die Pumpe zusätzlich oder anstelle der Druckdifferenz über den Rotor der erfasste Wert des Rotor-Drehmomentes und/oder ein absoluter Druck der Flüssigkeit vor oder hinter der Pumpe berücksichtigt wird.

Eine weitere vorteilhafte Ausgestaltung der Erfindung kann vorsehen, dass der Volumenstrom durch die Pumpe und/oder die Druckdifferenz über der Pumpe für einen Betriebszustand sowohl mit als auch ohne die Berücksichtigung eines oder mehrerer zusätzlicher Betriebsparameter oder aufgrund eines ersten und eines zweiten Satzes von Betriebsparametern ermittelt wird, dass die auf diese Weise ermittelten Werte verglichen und aus der Differenz eine Korrektur der Werte ermittelt wird.

Aus der Differenz, insbesondere den durch die beiden Ermittlungsverfahren ermittelten Druckdifferenzen über der Pumpe, können Störungen der ermittelten Werte detektiert werden. Beispielsweise lässt die Erfassung der axialen, auf den Rotor wirkenden Kraft eine direktere Ermittlung der Druckdifferenz zu als die theoretische Berechnung aus der Drehzahl und einem absoluten hydrostatischen Druckwert oder aus der Drehzahl und dem Drehmoment, das auf den Rotor wirkt. Durch die Differenz der Ergebnisse der beiden Ermittlungsmethoden kann auf Störungen oder Veränderungen in der Pumpe, beispielsweise bestimmte instationäre Betriebszustände oder Verstopfen, Verklebungen oder dergleichen, beispielsweise auch Verformungen des Pumpenrotors, geschlossen werden.

Das erfindungsgemäße Verfahren kann demnach vorsehen, dass die Druckdifferenz über der Pumpe einerseits durch die Erfassung einer axialen Kraft auf ein Rotorlager oder einer axialen Positionsverschiebung des Rotors gegen eine Lagerkraft und andererseits mittels der gemessenen Drehzahl des Rotors und eines gemessenen absoluten Drucks der Flüssigkeit vor oder hinter der Pumpe und/oder des auf den Rotor wirkenden Drehmomentes bestimmt wird und dass durch eine ermittelte Differenz zwischen den so bestimmten Werten der Druckdifferenz instabile Strömungszustände in der Pumpe oder geometrische Veränderungen, z. B. durch Ablagerungen, detektiert werden.

Die Erfindung bezieht sich nicht nur auf ein Betriebsverfahren, das sich verschiedener Ermittlungsmethoden der genannten Betriebsparameter der Pumpe je nach dem Bereich des Kennlinienfeldes bedient, in dem die Pumpe betrieben wird, sondern auch auf ein Verfahren zur Ermittlung der entsprechenden Kennlinien oder Kennlinienfelder. Insofern bezieht sich die Erfindung auf ein Verfahren zum Betrieb einer Pumpe mit einem Rotor zur Förderung von Flüssigkeiten, insbesondere einer Blutpumpe, bei dem der Volumenstrom durch die Pumpe und insbesondere die Druckdifferenz über der Pumpe ermittelt wird, wobei zur Ermittlung von Kennlinien der Pumpe die Drehzahl des Rotors, eine auf ein Axiallager des Rotors wirkende Kraft sowie ein absoluter Druck der Flüssigkeit vor oder hinter der Pumpe und/oder das auf den Rotor der Pumpe wirkende Drehmoment erfasst wird, das insbesondere mittels einer den Phasenstrom eines den Rotor antreibenden Elektromotors repräsentierenden Größe bestimmt wird.

Grundsätzlich werden bei der Ermittlung des Kennlinienfeldes somit mehr Betriebsparameter erfasst, als für die Ermittlung des Volumendurchsatzes der Pumpe und/oder der Druckdifferenz in einem bestimmten Kennlinienfeldbereich notwendig wäre. Durch die vielseitige Auswahl von Eingangsparametern für die Kennlinienfeldbestimmung wird der Einsatz unterschiedlicher Ermittlungsmethoden je nach dem Bereich des Kennlinienfeldes möglich, in dem der aktuelle Betriebszustand der Pumpe liegt.

Zudem bezieht sich die Erfindung auch auf eine Pumpe, die die oben beschriebenen Betriebsverfahren ermöglicht oder implementiert. Gegenstand der Erfindung ist somit unter anderem eine Pumpe mit einem Rotor zur Förderung von Flüssigkeiten, insbesondere einer Blutpumpe, mit einer Einrichtung zur Erfassung der Drehzahl des Rotors, einer Einrichtung zur Erfassung der Druckdifferenz über den Rotor, insbesondere mittels der Erfassung einer auf ein Axiallager des Rotors wirkenden Kraft, einer Einrichtung zur Erfassung eines absoluten Drucks am Einlass oder Auslass der Pumpe sowie insbesondere einer Einrichtung zur Erfassung des auf den Rotor wirkenden Drehmomentes, insbesondere durch Erfassung einer für den Phasenstrom eines den Rotor antreibenden Elektromotors (Phasenstrom eines BLDC-Motors) repräsentativen Größe.

Im Folgenden wird die Erfindung anhand von Ausführungsbeispielen in Figuren einer Zeichnung gezeigt und nachfolgend erläutert. Dabei zeigt
- Fig. 1: schematisch die Ansicht eines Patientenkörpers mit dem Herzen des Patienten und einer VAD (ventricular assist device)-Pumpe,
- Fig. 2: in einer dreidimensionalen Ansicht schematisch eine axiale Rotorpumpe,
- Fig. 3: in einer schematischen Seitenansicht das Gehäuse einer Axialpumpe mit einem Rotor, einem Antrieb und einem magnetischen Axiallager,
- Fig. 4: ein typisches Kennlinienfeld einer Pumpe mit Kennlinien, die eine Zuordnung einer Drehzahl und einer Druckdifferenz zu einem Volumenstrom ermöglichen, sowie
- Fig. 5: ein Diagramm gemäß Figur 4, anhand dessen eine Bestimmung der Druckdifferenz über der Pumpe anhand zwei verschiedener Verfahren und der Vergleich der Ergebnisse erläutert werden.

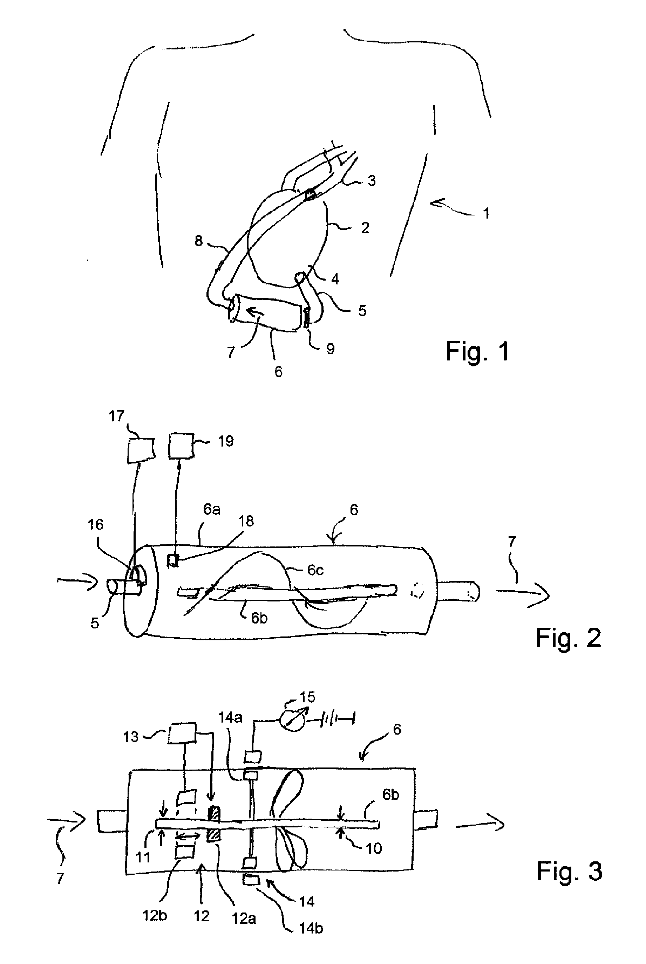

Figur 1 zeigt den Oberkörper eines Patienten 1 mit dem Herzen 2 des Patienten und einem Teil der Aorta 3. An einem Ventrikel 4 des Herzens ist ein Einlassstutzen 5 einer VAD-Pumpe 6 angeschlossen, die aus dem Ventrikel 4 in Richtung des Pfeils 7 Blut ansaugt und dieses über die Auslasskanüle 8 direkt in die Aorta 3 fördert.

Derartige Pumpen können die Pumpfunktion eines kranken bzw. nicht voll leistungsfähigen Herzens wesentlich unterstützen. Dies kann als vorübergehende Therapie oder auch als Dauertherapie angelegt sein. Als besonders vorteilhaft hat sich dabei die Verwendung von Axialrotorpumpen herausgestellt, die mittels eines schnell rotierenden Rotors im Pumpengehäuse in Axialrichtung 7 Blut fördern. Derartige Pumpen werden üblicherweise durch einen elektromotorischen Antrieb im Bereich des Pumpengehäuses angetrieben, der durch eine mitgeführte Batterie oder einen stationären Stromanschluss gespeist werden kann.

Um den Gesundheitszustand des Patienten sowie den Betriebszustand der Pumpe ausreichend genau kontrollieren zu können, ist es notwendig, den Volumendurchsatz durch die Pumpe zu ermitteln und zu verfolgen. Hierzu sind grundsätzlich verschiedene Verfahren bekannt, bei denen beispielsweise die Drehzahl des Rotors und die Druckdifferenz über den Rotor erfasst werden. Auch mittels der Drehzahl der Pumpe und der Messung des stationären Drucks des zu fördernden Blutes mittels eines Absolutdrucksensors 9 an der Pumpe ist die Ermittlung des Volumenstroms möglich. Schließlich kann der Volumendurchsatz auch mittels eines Ansatzes unter Berücksichtigung der Drehzahl des Pumpenrotors und des Drehmoments, das auf den Rotor wirkt, bestimmt werden.

Bei allen diesen Methoden wird üblicherweise aus den erfassten Messwerten für die Betriebsparameter Drehzahl des Rotors, Differenzdruck über den Rotor oder axiale Positionsverschiebung des Rotors gegen eine Lagerkraft, Drehmoment des Rotors, Absolutdruck im Bereich der Pumpe mittels Kennlinienfeldern der Volumenstrom und/oder der Differenzdruck über der Pumpe ermittelt.

In Figur 2 ist schematisch eine Axialpumpe 6 mit einem in einem Gehäuse 6a gelagerten Rotor 6b dargestellt, der ein oder mehrere Förderelemente 6c, beispielsweise in Form eines umlaufenden schraubenförmigen Förderblattes, aufweist. Durch Rotation des Rotors wird in dem Gehäuse 6a die zu fördernde Flüssigkeit bzw. das Blut in Richtung des Pfeils 7 gefördert.

In Figur 3 ist detaillierter die Lagerung und der Antrieb des Rotors der Pumpe 6 dargestellt. Zunächst ist im Bereich des Rotors 6b ein erstes Radiallager symbolisch dargestellt und mit dem Bezugszeichen 10 bezeichnet, während ein zweites Radiallager ebenso symbolisch dargestellt und mit 11 bezeichnet ist. Das zweite Radiallager 11 kann beispielsweise mit einem magnetischen Axiallager 12 kombiniert, jedoch auch von diesem separat aufgebaut sein. Das Axiallager 12 ist als regelbares magnetisches Axiallager ausgebildet, wobei ein erster ringförmiger Magnet 12a mit dem Rotor 6b verbunden ist und mit diesem rotiert.

Ein zweiter ringförmiger Magnet oder eine ringförmige Anordnung von Einzelmagneten 12b ist ortsfest im Gehäuse 6a der Pumpe 6 angeordnet und umgibt den Rotor 6b. Durch Abstoßung des ortsfesten Magneten 12b oder der ortsfesten Magnetanordnung 12b einerseits und des rotierenden Magneten 12a auf dem Rotor 6b wird eine auf den Rotor wirkende Axialkraft aufgenommen, die der Durchströmrichtung 7 der Flüssigkeit durch die Pumpe als Reaktionskraft entgegengesetzt ist.

Das Axiallager 12 weist einen Sensor zur Erfassung der axialen Position des auf der Nabe befestigten Magneten 12a auf, wobei die Information über die Axialposition an eine Steuerungseinrichtung 13 gemeldet wird. Damit kann die Regelschleife des Magnetlagers 12 geschlossen werden, und die Steuerungseinrichtung 13 kann die axiale Position des Magneten 12a und damit des Rotors 6b auf eine konstante Größe regeln. Die Kraft, die dabei durch das Axiallager aufgenommen wird, ist in einigen Fällen anhand der durch die Steuerungseinrichtung 13 aufgebrachten Stromstärke ermittelbar.

Die durch das Axiallager aufgenommene Kraft kann bei einem ungeregelten Lager beispielsweise durch Messung der axialen Auslenkung des Rotors gegen die Magnetkraft des Axiallagers bestimmt werden. Die Rotorposition kann mittels einer "Lagerspannung" ermittelt werden, welche aufgrund der Wirbelstromsensoren entsteht, die sensitiv bezüglich des Axialabstandes zu ortsfesten Magneten reagieren.

In Figur 3 ist zudem ein Elektromotor 14 dargestellt, der beispielsweise Permanentmagnete 14a, die mit dem Rotor fest verbunden sind, und einen Stator 14b, der mit dem Pumpengehäuse 6a verbunden ist, vorsehen kann. Durch entsprechende Ansteuerung von Statorwicklungen wird somit ein bürstenloser Elektromotor realisiert. Die Stromstärke, mit der der Stator 14b beaufschlagt wird, ist mittels eines Messgeräts 15 nachweisbar, und aus dieser Stromstärke kann in einfacher Weise das erzeugte Drehmoment auf den Rotor ermittelt werden.

Zurückkommend auf Figur 2 soll erwähnt werden, dass dort ein hydrostatischer Drucksensor 16 am Pumpeneinlassstutzen 5 dargestellt ist, der mit einer entsprechenden Verarbeitungseinrichtung 17 verbunden ist. Zudem ist in Figur 2 ein Temperatursensor 18 innerhalb des Pumpengehäuses 6a dargestellt, der ebenfalls mit einer Bearbeitungseinrichtung 19 verbunden ist. Der Temperatursensor 18 kann auch außerhalb der Pumpe in einem von der geförderten Flüssigkeit durchströmten Bereich angeordnet sein.

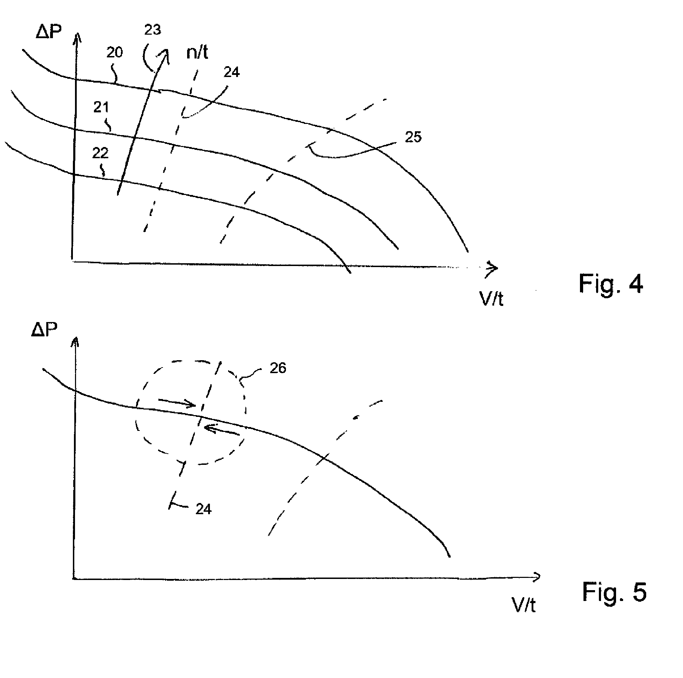

Figur 4 zeigt ein Diagramm, in dem auf der horizontalen Achse der Volumenstrom durch die Pumpe in Volumen pro Zeit gegen die Druckdifferenz über der Pumpe für verschiedene Drehzahlen der Pumpe aufgetragen ist. Dadurch ergibt sich ein Kennlinienfeld, in dem beispielsweise drei Kennlinien 20, 21, 22 für unterschiedliche Drehzahlen dargestellt sind. Der Pfeil 23 zeigt damit tendenziell die Übergangsrichtung zwischen Kurven verschiedener Drehzahlen an.

Damit werden in Form der Kennlinien Betriebspunkte der Pumpe (Druckdifferenz zwischen Ein- und Auslass der Pumpe sowie der durch die Pumpe geförderte Volumenstrom) für verschiedene Drehzahlen der Pumpe dargestellt. Dabei wird zur Kennlinienbestimmung jeweils die Drehzahl und die axiale Lagerposition des Rotors als repräsentative Größe für die Druckdifferenz über den Rotor aufgenommen. Durch entsprechende Regressionskurven können Messpunkte zu Kennlinien interpoliert werden. Im Betrieb der Pumpe kann somit aus der Drehzahl und der axialen Lagerposition des Rotors beispielsweise der Volumenstrom durch die Pumpe bestimmt werden.

Da erkannt wurde, dass für bestimmte Pumpentypen Bereiche im Kennlinienfeld existieren, in denen die Zuordnung zwischen der Lagerposition/Druckdifferenz und dem Volumenstrom bei bekannter Drehzahl nicht eindeutig oder zumindest unscharf ist, wurde für bestimmte Bereiche des Kennlinienfeldes ein anderes Verfahren gewählt, um den Volumenstrom zu bestimmen. In Abhängigkeit vom Bereich des Kennlinienfeldes, in dem sich der Betriebspunkt der Pumpe befindet, also beispielsweise in Abhängigkeit von der Drehzahl und/oder der Druckdifferenz über der Pumpe, kann somit das entsprechende Ermittlungsverfahren gewählt werden.

In Figur 4 ist beispielsweise zwischen den beiden gestrichelten Linien 24 und 25 ein Teilfeld des Kennlinienfeldes gebildet, in dem der Volumenstrom nicht aus der Drehzahl und der Druckdifferenz über den Rotor oder zumindest nicht aus diesen Parametern allein bestimmt wird. In dem Feld zwischen den Linien 24 und 25 kann beispielsweise das Drehmoment des Rotors, ermittelt durch den Strom des elektrischen Antriebsmotors des Rotors, und/oder der Absolutdruck am Einlass oder Auslass der Pumpe berücksichtigt werden. Diese Größen können allein oder in Kombination mit der Drehzahl ausgewertet werden, es kann jedoch zudem auch die Lagerposition als Indikator für die Druckdifferenz über den Rotor in die Bestimmung des Volumenstroms mit einbezogen werden.

Figur 5 zeigt die Verwendung mehrerer Messverfahren in einem Grenzbereich/Übergangsbereich des Kennlinienfeldes, der durch den gestrichelten Kreis 26 angedeutet sein soll. Dieser Bereich liegt in der Nachbarschaft der Trennlinie 24 zwischen den beiden Bereichen des Kennlinienfeldes, die verschiedene Messmethoden zur Ermittlung des Volumenstroms erfordern. Im Bereich um die Trennlinie 24 herum kann dann beispielsweise der Volumenstrom oder die Druckdifferenz über der Pumpe durch verschiedene Verfahren unter Verwendung unterschiedlicher erfasster Betriebsparameter ermittelt werden. Sind die Werte beispielsweise der so ermittelten Druckdifferenz über der Pumpe unterschiedlich, so kann hieraus auf Veränderungen in der Pumpe, beispielsweise durch Verklebungen, Verstopfungen, Verformungen oder instabile Strömungszustände, hingewiesen werden. Die Detektion derartiger Phänomene ist beim Betrieb einer Blutpumpe besonders wichtig, um Gefährdungen des Patienten auszuschließen.

Die Erfindung bezieht sich außer auf ein Verfahren zum Betrieb einer derartigen Pumpe und zur Bestimmung des Volumenstroms in verschiedenen Kennlinienfeldbereichen auch auf ein Verfahren zur Ermittlung eines entsprechenden Kennlinienfeldes unter Verwendung verschiedener Betriebsparameter in unterschiedlichen Bereichen des Kennlinienfeldes.

Zudem bezieht sich die Erfindung auch auf eine Pumpe, die entsprechende Einrichtungen zur Erfassung der notwendigen Betriebsparameter aufweist.

Durch die Erfindung wird die Ermittlung des Volumenstroms durch eine Pumpe erheblich verbessert, insbesondere für solche Pumpen, für die ein einziges Messverfahren bzw. die Orientierung an bestimmten Kennlinien über das gesamte Kennlinienfeld aus konstruktiven Gründen nicht ausreicht.

## Patentansprüche

1. Verfahren zum Betrieb einer Pumpe (6) mit einem Rotor (6b, 6c) zur Förderung von Flüssigkeiten, bei dem der Volumenstrom durch die Pumpe und insbesondere die Druckdifferenz über der Pumpe ermittelt wird,
wobei wenigstens ein erster und insbesondere ein zweiter Betriebsparameter einer ersten Gruppe von Betriebsparametern erfasst und in Abhängigkeit von dem Wert des ersten und insbesondere auch dem Wert eines zweiten Betriebsparameters der ersten Gruppe der Volumenstrom durch die Pumpe und insbesondere die Druckdifferenz aus erfassten Werten der Betriebsparameter entweder aus der ersten Gruppe von Betriebsparametern ermittelt wird oder zur Ermittlung des Volumenstroms und insbesondere der Druckdifferenz Werte eines anderen Satzes von Betriebsparametern, insbesondere wenigstens eines zusätzlichen Betriebsparameters, berücksichtigt werden.

2. Verfahren nach Anspruch 1, **dadurch gekennzeichnet, dass** die erste Gruppe von Betriebsparametern die erfasste Drehzahl der Pumpe enthält.

3. Verfahren nach Anspruch 1 oder 2, **dadurch gekennzeichnet, dass** die erste Gruppe von Betriebsparametern die erfasste Druckdifferenz über den Rotor enthält.

4. Verfahren nach Anspruch 1, 2 oder 3, **dadurch gekennzeichnet, dass** die erste Gruppe von Betriebsparametern die erfasste Kraft auf ein Rotorlager (12, 12a, 12b), insbesondere ein Axiallager der Pumpe (6), enthält.

5. Verfahren nach Anspruch 1 oder einem der folgenden, **dadurch gekennzeichnet, dass** die erste Gruppe von Betriebsparametern enthält: die Temperatur der Flüssigkeit und/oder den absoluten Druck der Flüssigkeit vor oder hinter der Pumpe und/oder das auf den Rotor der Pumpe wirkende Drehmoment, das insbesondere mittels einer den Phasenstrom eines den Rotor antreibenden Elektromotors, insbesondere BLDC-Motors (bürstenlosen DC-Motors), repräsentierenden Größe bestimmt wird.

6. Verfahren nach Anspruch 1 oder einem der folgenden, **dadurch gekennzeichnet, dass** ein zusätzlicher Parameter durch den absoluten Druck der Flüssigkeit vor oder hinter der Pumpe und/oder das auf den Rotor der Pumpe wirkende Drehmoment gebildet ist, das insbesondere mittels einer den Phasenstrom eines den Rotor antreibenden Elektromotors repräsentierenden Größe bestimmt wird.

7. Verfahren nach Anspruch 1, **dadurch gekennzeichnet, dass** die Druckdifferenz über dem Rotor (6) (insbesondere repräsentiert durch die erfasste Lagerposition) und die Drehzahl der Pumpe erfasst und in Abhängigkeit von wenigstens einem der erfassten Werte der Volumenstrom durch die Pumpe entweder aus den erfassten Werten dieser beiden Betriebsparameter ermittelt wird oder zur Ermittlung des Volumenstroms und insbesondere der Druckdifferenz über die Pumpe zusätzlich oder anstelle der Druckdifferenz über den Rotor der erfasste Wert des Rotor-Drehmomentes und/oder ein absoluter Druck der Flüssigkeit vor oder hinter der Pumpe berücksichtigt wird.

8. Verfahren nach Anspruch 1 oder einem der folgenden, **dadurch gekennzeichnet, dass** der Volumenstrom durch die Pumpe (6) und/oder die Druckdifferenz über der Pumpe für einen Betriebszustand sowohl mit als auch ohne die Berücksichtigung eines oder mehrerer zusätzlicher Betriebsparameter ermittelt wird, dass die auf diese Weise ermittelten Werte verglichen und aus der Differenz eine Korrektur der Werte ermittelt wird.

9. Verfahren nach Anspruch 8, **dadurch gekennzeichnet, dass** die Druckdifferenz über der Pumpe einerseits durch die Erfassung einer axialen Kraft auf ein Rotorlager (12, 12a, 12b) oder einer axialen Positionsverschiebung des Rotors gegen eine Kraft bestimmt und andererseits mittels der gemessenen Drehzahl des Rotors (6b, 6c) und eines gemessenen Drucks der Flüssigkeit vor oder hinter der Pumpe und/oder des auf den Rotor (6b, 6c) wirkenden Drehmomentes bestimmt wird und dass durch eine ermittelte Differenz zwischen den so bestimmten Werten der Druckdifferenz instabile Strömungszustände in der Pumpe oder geometrische Veränderungen z.B. durch Ablagerungen detektiert werden.

10. Verfahren zum Betrieb einer Pumpe (6) mit einem Rotor (6b, 6c), insbesondere einer Blutpumpe, zur Förderung von Flüssigkeiten, bei dem der Volumenstrom durch die Pumpe und insbesondere die Druckdifferenz über der Pumpe ermittelt wird,
wobei zur Ermittlung von Kennlinien der Pumpe die Drehzahl des Rotors, eine auf ein Axiallager (12, 12a, 12b) des Rotors wirkende Kraft oder eine axiale Positionsverschiebung des Rotors gegen eine Kraft sowie ein absoluter Druck der Flüssigkeit vor oder hinter der Pumpe und/oder das auf den Rotor der Pumpe wirkende Drehmoment erfasst wird, das insbesondere mittels einer den Speisestrom eines den Rotor antreibenden Elektromotors repräsentierenden Größe bestimmt wird.

11. Pumpe (6) mit einem Rotor (6b, 6c) zur Förderung von Flüssigkeiten, insbesondere Blutpumpe, mit einer Einrichtung zur Erfassung der Drehzahl des Rotors, einer Einrichtung zur Erfassung der Druckdifferenz über den Rotor, insbesondere mittels der Erfassung einer auf ein Axiallager (12, 12a, 12b) des Rotors wirkenden Kraft oder einer axialen Positionsverschiebung des Rotors gegen eine Kraft, einer Einrichtung (9) zur Erfassung eines hydrostatischen Drucks am Einlass oder Auslass der Pumpe sowie insbesondere einer Einrichtung (14) zur Erfassung des auf den Rotor wirkenden Drehmomentes, insbesondere durch Erfassung einer für den Speisestrom eines den Rotor antreibenden Elektromotors repräsentativen Größe.

12. Pumpe gemäß Anspruch 11, **gekennzeichnet durch** eine Einrichtung, die in Abhängigkeit von wenigstens einem ersten erfassten Betriebsparameter einer ersten Gruppe von Betriebsparametern den Volumenstrom **durch** die Pumpe und insbesondere die Druckdifferenz aus erfassten Werten der Betriebsparameter entweder aus der ersten Gruppe von Betriebsparametern oder aus einer zweiten Gruppe von Betriebsparametern ermittelt.

13. Pumpe gemäß Anspruch 11 oder 12, **dadurch gekennzeichnet, dass** die erste Gruppe von Betriebsparametern die erfasste Drehzahl der Pumpe enthält.

14. Pumpe gemäß Anspruch 11, 12 oder 13, **dadurch gekennzeichnet, dass** die erste Gruppe von Betriebsparametern die erfasste Druckdifferenz über dem Rotor enthält.

15. Pumpe gemäß Anspruch 11, 12, 13 oder 14, **dadurch gekennzeichnet, dass** die erste Gruppe von Betriebsparametern enthält: die Temperatur der Flüssigkeit und/oder den absoluten Druck der Flüssigkeit vor oder hinter der Pumpe und/oder das auf den Rotor der Pumpe wirkende Drehmoment, das insbesondere mittels einer den Phasenstrom eines den Rotor antreibenden Elektromotors, insbesondere BLDC-Motors, repräsentierenden Größe bestimmt wird.
